# EUROPEAN PATENT APPLICATION

(11) **EP 4 000 514 A1**
(43) Date of publication of application: **25.05.2022**
(21) Application number: 20207218.7
(22) Date of filing: 12.11.2020
(51) Int. Cl.: A61B 5/145, A61B 5/1486, G01N 27/30, G01N 27/327

(54) **METHOD FOR PRODUCING AN ANALYTE SENSOR, AN ANALYTE SENSOR, AND A USE THEREOF**

(71) Applicant: Roche Diabetes Care GmbH, 68305 Mannheim (DE); F. Hoffmann-La Roche AG, 4070 Basel (CH)
(72) Inventor: Sliozberg, Kirill, 68305 Mannheim (DE); Steck, Alexander, 68305 Mannheim (DE)
(74) Representative: Altmann Stößel Dick Patentanwälte PartG mbB

(57) **Abstract**

A method (110) for producing an analyte sensor (112), an analyte sensor (112) obtainable by this method (110), and a use of the analyte sensor (112) are disclosed. The method (110) comprising the steps of:
a) providing a first substrate (114) having
∘ a first side (116), and
∘ a second side (118), wherein the second side (118) has a first layer (120) comprising a first conductive material (122);

b) providing a second substrate (124) having
∘ a first side (126), wherein the first side (126) has a second layer (130) comprising a second conductive material (132), and
∘ a second side (128), wherein second side (128) has a third layer (134) comprising a third conductive material (136);

c) applying a layer (138) of an conductive preparation (140) onto at least one of the first side (116) of the first substrate (114) or the third layer (134) or a portion thereof, wherein the conductive preparation (140) comprises
∘ a plurality of conductive particles, and
∘ at least one polymeric binder;

d) laminating the first side (116) of the first substrate (114) with the second side (128) of the second substrate (124); and
e) obtaining the analyte sensor (112).

The method (110) is a cost-efficient process which allows producing the analyte sensor (112) in a reliable and fast fashion, whereby an individual tailoring of the analyte sensor (112) is, concurrently, possible.

## Description

### Field of the invention

The present invention relates to a method for producing an analyte sensor, to an analyte sensor and to a use thereof. The analyte sensor may, primarily, be used for a long-term monitoring of at least one analyte concentration in a body fluid, in particular of a blood glucose level or of a concentration of one or more other analytes in the body fluid. The invention may be applied both in the field of home care as well as in the filed of professional care, such as in hospitals. However, other applications are feasible.

### Related art

Monitoring body functions, in particular monitoring one or more concentrations of certain analytes, plays an important role in the prevention and treatment of various diseases. Without restricting further possible applications, the invention is described in the following with reference to glucose monitoring in an interstitial fluid. However, the invention can also be applied to other types of analytes. Blood glucose monitoring may, specifically, be performed by using electrochemical analyte sensors besides optical measurements. Examples of electrochemical analyte sensors for measuring glucose, specifically in blood or other body fluids, are known from US 5,413,690 A, US 5,762,770 A, US 5,798,031 A, US 6,129,823 A or US 2005/0013731 A1.

In the recent past, continuous measuring of glucose in the interstitial tissue, also referred to as "continuous glucose monitoring" or abbreviated to "CGM", has been established as important method for managing, monitoring, and controlling a diabetes state. Herein, an active sensor region is applied directly to a measurement site which is, generally, arranged in an interstitial tissue, and may, for example, convert glucose into an electrically charged entity by using an enzyme, in particular glucose oxidase (GOD) and/or glucose dehydrogenase (GDH). As a result, the detectable charge may be related to the glucose concentration and can, thus, be used as a measurement variable. Examples thereof are described in US 6,360,888 B1 or US 2008/ 0242962 A1.

Typically, current continuous monitoring systems are transcutaneous systems or subcutaneous systems. Accordingly, the analyte sensor or at least a measuring portion of the analyte sensor may be arranged under the skin of the user. However, an evaluation and control part of the system, which may also be referred to as a "patch", may, generally, be located outside of the body of a user. Herein, the analyte sensor is generally applied by using an insertion instrument, which is, in an exemplary fashion, described in US 6,360,888 B1. However, other types of insertion instruments are also known. Further, the evaluation and control part may, typically, be required which may be located outside the body tissue and which has to be in communication with the analyte sensor. Generally, communication is established by providing at least one electrical contact between the analyte sensor and the evaluation and control part, wherein the contact may be a permanent or a releasable electrical contact. Other techniques for providing electrical contacts, such as by appropriate spring contacts, are generally known and may also be applied.

In continuous glucose measuring systems, the concentration of the analyte glucose may be determined by employing an analyte sensor comprising an electrochemical cell having at least two individual electrodes. In particular, the analyte sensor may comprise two individual electrodes, i.e. a working electrode and a combined counter/reference electrode. As an alternative, the analyte sensor may comprise three individual electrodes, i.e. a working electrode, a counter electrode and a reference electrode. Alternatively, the three individual electrodes may be two working electrodes and a combined counter/reference electrode. As a further alternative, the analyte sensor may comprise at least four electrodes, i.e. at least two individual working electrodes, an individual counter electrode and an individual reference electrode, or a combined counter/reference electrode. Herein, the at least one working electrode may have a reagent layer comprising an enzyme with a redox active enzyme co-factor adapted to support an oxidation of the analyte in the body fluid. During catalyzing the glucose oxidation, a reduction of the enzyme occurs, thus producing a reduced enzyme. Thereafter, the reduced enzyme is being directly or indirectly electrochemically oxidized. Hereby, at least one electrode signal is generated from which the analyte concentration can be determined. Further, the analyte sensor may comprise at least one contact for each electrode, especially, for establishing an electrical contact between each electrode and the evaluation and control part.

In an embodiment in which the analyte sensor comprises at least one third electrode, using a flat double-sided substrate, however, results in structuring at least one side of the substrate in a fashion that at least two electrodes can be placed there. As an alternative, stacked arrangements have been proposed in which the electrodes are distributed over more than a single substrate.

US 10,321,863 B2 discloses analyte sensor connectors which connect analyte sensors, e.g., conductive members of analyte sensors, to other devices such as sensor electronics units, e.g., sensor control units. Further disclosed are systems which include analyte sensors, analyte sensor connectors, and analyte sensor electronics units, as well as methods of establishing and maintaining connections between analyte sensors and analyte sensor electronics units, and methods of analyte monitoring/detection. Further disclosed are methods of making analyte sensor connectors and systems which include analyte sensor connectors.

US 10,349,873 B2 discloses systems and methods of use for continuous analyte measurement of a host's vascular system. In some embodiments, a continuous glucose measurement system includes a vascular access device, a sensor and sensor electronics, the system being configured for insertion into communication with a host's circulatory system.

### Problem to be solved

It is therefore an objective of the present invention to provide a method for producing an analyte sensor, an analyte sensor and a use thereof, which at least partially avoid the shortcomings of known analyte sensors and related methods and which at least partially address the above-mentioned challenges.

In particular, it is desired that the method for producing an analyte sensor is a cost-efficient process which allows producing the analyte sensor in a reliable and fast fashion, whereby an individual tailoring of the analyte sensor may, concurrently, be possible.

### Summary of the invention

This problem is solved by a method for producing an analyte sensor, an analyte sensor and a use thereof having the features of the independent claims. Preferred embodiments of the invention, which may be implemented in an isolated way or in any arbitrary combination, are disclosed in the dependent claims and throughout the specification.

In a first aspect of the present invention, a method for producing an analyte sensor is disclosed. The method comprises the following steps of:
a) providing a first substrate having
   ∘ a first side, and
   ∘ a second side, wherein the second side has a first layer comprising a first conductive material;
b) providing a second substrate having
   ∘ a first side, wherein the first side has a second layer comprising a second conductive material, and
   ∘ a second side, wherein second side has a third layer comprising a third conductive material;
c) applying a layer of an conductive preparation onto at least one of the first side of the first substrate or the third layer or a portion thereof, wherein the conductive preparation comprises
   ∘ a plurality of conductive particles, and
   ∘ at least one polymeric binder;
d) laminating the first side of the first substrate with the second side of the second substrate; and
e) obtaining the analyte sensor.

Herein, the indicated steps may, preferably, be performed in the given order, whereby, in particular, the order of steps a) and b) can be exchanged without altering the result of the method. Further, depending on the selected procedure in step c), steps b) and c) may be performed prior to step a), or steps a) and c) may be performed prior to step b). Further, consecutive steps may, in particular, be performed in an overlapping fashion. Further, additional steps, whether described herein or not, may be performed, too.

As generally used, the term "analyte sensor" refers to an arbitrary device being configured to perform a detection of an analyte by acquiring at least one measurement signal for conducting, especially to perform at least one medical analysis. As particularly preferred, the analyte sensor may be a fully or partially implantable analyte sensor which may, particularly, be adapted for performing the detection of the analyte in a body fluid of a user in a subcutaneous tissue, particularly in an interstitial fluid. As used herein, the terms "implantable analyte sensor" or "transcutaneous analyte sensor" refer to an arbitrary analyte sensor being adapted to be fully or at least partly arranged within the body tissue of the patient or the user. For this purpose, the analyte sensor may comprise an insertable portion. Herein, the term "insertable portion" generally refers to a part or component of the analyte sensor configured to be insertable into an arbitrary body tissue. Other parts or components of the analyte sensor, in particular a patch, may remain outside of the body tissue. Herein, the term "patch" generally refers to a further part or component of the analyte sensor which is configured to arrange the insertable portion at a desired location at the body of the user and to, concurrently, provide an interface to a separate electronics unit.

As generally used, the term "electronics unit" refers to a separate device outside the analyte sensor which is configured to control and evaluate the electrodes of the analyte sensor, in particular by providing electronic voltages and/or electronic currents to the electrodes, by receiving electronic voltages and/or electronic currents from the electrodes, and by evaluating the received electronic voltages and/or electronic currents from the electrodes. Herein, the electronics unit may communicate with electrically conducting contact pads which are comprised by the patch in a wire-bound or in a wireless fashion.

With particular respect to the present invention, the insertable portion of the analyte sensor may comprise the first substrate having a first electrode, the second substrate having a second electrode, and the layer of the conductive preparation which laminates the first side of the first substrate with the second side of the second substrate in a fashion that a third interjacent electrode is formed. For a purpose of controlling and evaluating the electrodes, each electrode can be arranged in a fashion that it may extend to the corresponding electrically conducting contact pad as comprised by the patch. As an alternative, the analyte sensor may, further, comprise conductive traces configured to provide a desired electrical contact between each electrode and the corresponding contact pad.

As further generally used, both terms "user" and "patient" refer to a human being or an animal, independent from the fact that the human being or animal, respectively, may be in a healthy condition or may suffer from one or more diseases. As an example, the user or the patient may be a human being or an animal suffering from diabetes. However, additionally or alternatively, the invention may be applied to other types of users, patients or diseases.

As further used herein, the term "body fluid", generally, refers to a fluid, in particular a liquid, which is typically present in a body or a body tissue of the user or the patient and/or may be produced by the body of the user or the patient. Preferably, the body fluid may be selected from the group consisting of blood and interstitial fluid. However, additionally or alternatively, one or more other types of body fluids may be used, such as saliva, tear fluid, urine or other body fluids. During the detection of the at least one analyte, the body fluid may be present within the body or body tissue. Thus, the analyte sensor may, specifically, be configured for detecting the at least one analyte within the body tissue.

As further used herein, the term "analyte" refers to an arbitrary element, component, or compound being present in the body fluid, wherein the presence and/or the concentration of the analyte may be of interest to the user, the patient, to medical staff, such as a medical doctor. In particular, the analyte may be or may comprise at least one arbitrary chemical substance or chemical compound which may participate in the metabolism of the user or the patient, such as at least one metabolite. As an example, the at least one analyte may be selected from the group consisting of glucose, cholesterol, triglycerides, lactate, ketones urea, creatinine, glutamate, ethanol, ascorbic acid, choline, acetylcholine, polyphenol, monophenols, dihydroxyphenols, Bisphenol A (BPA) and hydrochlorothiazide (Hct). Additionally or alternatively, however, other types of analytes may be used and/or any combination of analytes may be determined, such as a concentration of an electrolyte, or a pO₂, pCO₂, pH, or Hb value, which indicates a concentration of oxygen, carbon dioxide, hydrogen, or hemoglobin, respectively. The detection of the at least one analyte by the analyte sensor may, in particular, be an analyte-specific detection. As an alternative, the analyte sensor may be configured to specifically detect at least two analytes in a concurrent or consecutive manner. By way of example, the analyte sensor may be configured to detect glucose and, concurrently or consecutively, a further analyte, preferably selected from lactate or ketones, in a specific fashion. However, without restricting further possible applications, the present invention is described herein with particular reference to a monitoring of glucose in an interstitial fluid.

In particular, the analyte sensor may be an electrochemical sensor. As used herein, the term "electrochemical sensor" refers to an analyte sensor which is adapted for a detection of an electrochemically detectable property of the analyte, such as an electrochemical detection reaction. Thus, for example, the electrochemical detection reaction may be detected by applying and comparing one or more electrode potentials. Specifically, the electrochemical sensor may be adapted to generate the at least one measurement signal which may, directly or indirectly, indicate a presence and/or an extent of the electrochemical detection reaction, such as at least one current signal and/or at least one voltage signal. The measurement may be a qualitative and/or a quantitative measurement. Still, other embodiments are feasible.

The electrochemical sensor as used herein is arranged in a fashion of an electrochemical cell and, thus, employs at least one three individual electrodes. As generally used, the term "electrode" refers to an entity of the test element which is adapted to contact the body fluid, either directly or via at least one semi-permeable membrane or layer. Herein, each electrode comprises an electrically conductive material. As generally used, the terms "electrically conductive material" or simply "conductive material" refers to a substance which is designed for conducting an electrical current through the substance. For this purpose, a highly conductive material having a low electrical resistance is preferred, in particular to avoid a dissipation of electrical energy carried by the electrical current within the substance. Preferably, the conductive material may be selected from a noble metal, especially gold; or from an electrically conductive carbon material; however, further kinds of conductive materials may also be feasible. Further, as already indicated above, at least one electrode, in particular at last one working electrode, may have a reagent layer comprising an enzyme with a redox active enzyme co-factor adapted to support an oxidation of the analyte in the body fluid.

Each electrode may be embodied in a fashion that an electrochemical reaction may occur at at least one surface of the electrode. In particular, the electrodes may be embodied in a manner that oxidative processes and/or reductive processes may take place at selected surfaces of the electrodes. Generally, the term "oxidative process" refers to a first chemical or biochemical reaction during which an electron is released from a first substance, such an atom, an ion, or a molecule, which is oxidized thereby. A further chemical or biochemical reaction by which a further substance may accept the released electron is, generally, denominated by the term "reductive process". Together, the first reaction and the further reaction may also be denominated as a "redox reaction". As a result, an electrical current, which relates to moving electrical charges, may be generated hereby. Herein, a detailed course of the redox reaction may be influenced by an application of an electrical potential.

As further used herein, the term "determining" or any grammatical variation thereof relates to a process of generating at least one representative result, in particular, by evaluating the at least one measurement signal as acquired by the analyte sensor. Further, the term "evaluating" or any grammatical variation thereof refers to an application of methods for displaying the at least one measurement signal and deriving the at least one representative result therefrom. The at least one measurement signal may, specifically, be or comprise at least one electronic signal, such as at least one voltage signal and/or at least one current signal. The at least one signal may be or may comprise at least one analogue signal and/or may be or may comprise at least one digital signal. Especially in electrical systems, it may be required to apply a prespecified signal to a specific device in order to be able to record the desired measurement signal. By way of example, measuring a current signal may require the application of a voltage signal to the device, or vice-versa.

As further used herein, the term "monitoring" or any grammatical variation thereof refers to a process of continuously acquiring data and deriving desired information therefrom without user interaction. For this purpose, a plurality of measurement signals are generated and evaluated, wherefrom the desired information is determined. Herein, the plurality of measurement signals may be recorded within fixed or variable time intervals or, alternatively or in addition, at an occurrence of at least one prespecified event. In particular, the analyte sensor as used herein may, especially, be configured for a continuous monitoring of one or more analytes, in particular of glucose, such as for managing, monitoring, and controlling a diabetes state.

According to step a) of the present invention, a first substrate is provided while according to step b), a second substrate is provided. It is emphasized here that, as indicated above, the sequential processing of steps a) and b) may be exchanged without having any effect on the analyte sensor as obtained by the present method. As used herein, the terms "first", "second" and "third" are considered as description without specifying an order and without excluding a possibility that other elements of that kind may be present. As used herein, the term "substrate" refers to an arbitrary element having a substrate body which is designed to carry one or more other elements disposed thereon or therein. As particularly preferred, the substrate may be a planar substrate. As generally used, the term "planar" refers to a body comprising extensions in two dimensions, typically denoted as "surface" of the substrate, which exceed the extension in a third dimension, usually denoted as "thickness" of the substrate, by a factor of 2, at least a factor of 5, at least a factor of 10, or even at least a factor of 20 or more. The substrate may, specifically, have an elongated shape, such as a strip shape or a bar shape; however, other kinds of shapes may also be feasible. As generally used, the term "elongated shape" indicates that each surface of the planar body has an extension in a direction along the elongation which exceeds an extension perpendicular hereto by at least a factor of 2, at least a factor of 5, at least a factor of 10, or even at least a factor of 20 or more. Preferably, the substrate may have a lateral extension of 1 mm to 500 mm, preferably of 10 mm to 100 mm, more preferred of 20 mm to 50 mm, and a thickness of 10 µm to 100 µm, preferably of 20 µm to 80 µm, more preferred of 50 µm to 80 µm; however, a further lateral extension and/or thickness may also be feasible, in particular in an embodiment in which reel-to-reel manufacturing is applied to a potentially endless substrate.

The substrate may comprise at least partially, preferably completely, at least one electrically insulating material. As generally used, the terms "electrically insulating" or simply "insulating" relate to a type of material which is not capable of carrying an electrical current. By way of example, the electrically insulating material may be selected from polyethylene terephthalate (PET), polycarbonate (PC), polytetrafluoroethylene (PTFE), polyimide (PI), acrylonitrile butadiene styrene (ABS), styrene-acrylonitrile resin (SAN), acrylonitrile styrene acrylate (ASA), styrene butadiene copolymer (SB), polystyrene (PS), polyethlene (PE), ethylene-vinyl acetate (EVA), polypropylene (PP), polybutylene (PB), polyisobutene (PIB), poylvinylchloride (PVC), polyvinyl alcohol (PVAL), polylactic acid (PLA), polyamide (PA), polymethylmethacrylate (PMMA), polyoxymethylene (POM), polyphenylene sulfide (PPS), polysulfone (PSU), polyvinylidene fluoride (PVDF), fluorinated ethylene propylene (FEP), ethylene tetrafluoroethylene (ETFE), polyvinyl fluoride (PVF), polyethersilfone (PES), polyether ether ketone (PEEK,) perfluoroalkoxy alkane (PFA), polyetherketoneetherketoneketone (PEKEKK); however, other kinds of electrically insulating materials may also be feasible. This particular arrangement of the substrate allows avoiding unwanted currents between electrically conducting elements carried by the substrate.

In accordance with steps a) and b), each of the first substrate and of the second substrate has a first side and a second side. As already indicated above, the terms "first" and "second" are considered as description without specifying an order with respect to both sides of the first substrate. As generally used, the term "side" refers to a surface of the substrate. In a particularly preferred arrangement, the first side and the second side of the substrate may constitute opposing sides of the substrate. As generally used, the term "opposing sides" refers to the two planar surfaces of the flat substrate.

While the second side of the first substrate has a first layer which comprises a first conductive material; the first side of the first substrate may, preferably, be blank, or may, as an alternative, comprise at least one electrically insulating layer. As described below in more detail, the first layer may be, comprise, or assume a function of a first electrode. As generally used, the term "blank" refers to an uncoated insulating surface. For the terms "conductive material", "electrode", "insulating", reference can be made to the definitions given above. As used herein, the term "layer" refers to a volume comprising a material having extensions in two lateral dimensions, typically denoted as "lateral extension" or simply "extension" of the layer, which exceed the extension in a third dimension, usually denoted as "thickness" of the layer, by a factor of 2, at least a factor of 5, at least a factor of 10, or even at least a factor of 20 or more, wherein the layer may be carried by the respective substrate, in particular, in order to provide stability and integrity to the layer. The layer may, specifically, have an elongated shape, such as a strip shape or a bar shape; however, other kinds of shapes may also be feasible. In general, the layer may, partially or completely, cover a respective side of the substrate. In a preferred embodiment, in which the layer may only partially cover a portion of the respective side of the substrate, an insulating layer may, partially or completely, cover the remaining portion of the substrate. In general, the layer may, preferably, be produced in an additive process by applying the desired material to the selected substrate; however, a further process of producing the layer may also be feasible. In particular, the additive process may be selected from at least one process as indicated below, wherein sputtering may particularly be preferred. For further details, reference can be made to the description below, especially in connection with step c).

Further, the first side of the second substrate has a second layer which comprises a second conductive material while the second side of the second substrate has a third layer which comprises a third conductive material. For the terms "first", "second", "third", "layer" and "conductive material" reference can be made to the definitions given above. Herein, each of the second layer and the third layer may be, comprise, or assume a function of a second electrode or a third electrode, respectively. In this particularly preferred arrangement, the present invention solves the problem of providing an analyte sensor comprising three electrodes in fashion that the first electrode, as described above or below, is comprised by the first substrate while the second electrode and the third electrode are comprised by the second substrate are not located within a common plane of the second substrate.

According to step c), a layer of a conductive preparation is applied onto the third layer or a portion thereof. As an alternative or in addition, the layer of the conductive preparation is applied onto the first side of the first substrate or a portion thereof. It is emphasized here that selecting either procedure may not result in a difference in the analyte sensor as obtained by the present method. As generally used, the term "preparation" refers to a substance which comprises at least two different components, i.e. at least one first component and at least one second component. For the term "conductive" reference may be made to the definition given above. Herein, the at least one first component is or comprises a plurality of conductive particles, wherein the at least one second component is or comprises at least one polymeric binder. However, further types of components may also be conceivable, in particular at least one solvent. As generally used, the term "solvent" refers to at least one fluidic component which may, preferably, be a volatile component. As further generally used, the term "volatile" relates to a component which is capable of spontaneously leaving the preparation after application, preferably, within a limited period of time. The at least one solvent may, in particular, be used for improving the applying of the preparation but may, after application, be dispensable.

As generally used, the term "particle" refers to a kind of material which has a diameter of 1 nm to 1 mm, preferred 10 nm to 100 µm, in particular of 1 µm to 50 µm. As generally used, the term "diameter" of a particle relates to a typical extension through the body of the diameter. In a particularly preferred embodiment, the conductive particles may be or comprise carbon, Ag, AgCl or Ag/AgCl.

As further generally used, the term "binder" refers to a further substance designated to maintain the plurality of the conductive particles within the preparation at least partially, preferably completely, together. The conductive particles may, in particular, be dispersed within the at least one binder. Especially, the conductive particles may be homogeneously dispersed within the at least one binder. Herein, the at least one polymeric binder may be selected from at least one of a thermoplastic polyurethane (TPU), thermoplastic polyolefin (TPO), thermoplastic vulcanizates (TPV), thermoplastic copolyester (TPC), thermoplastic polyamides (TPA), a styrenic block copolymer (TPS), a copolymer of vinyl chloride (VC) and vinyl acetate (VAc) (Vinylester or vinylether), polyvinylether, polyvinylester, an acrylate, a methacrylate , an acrylic resin, a styrene acrylic resin, a vinyl acetal, a polyester, a polyether, acrylonitrile butadiene styrene (ABS), styrene-acrylonitrile resin (SAN), acrylonitrile styrene acrylate (ASA), styrene butadiene copolymer (SB), polystyrene (PS), polyethlene (PE), ethylene-vinyl acetate (EVA), polypropylene (PP), polybutylene (PB), polyisobutene (PIB), poylvinylchloride (PVC), polyvinyl alcohol (PVAL), polylactic acid (PLA). As used herein, the term "applying" or any grammatical variation thereof refers to a process of depositing the at least one material used for generating the layer on a substrate. The process of depositing may, preferably, be selected from at least one of inkjet printing, gravure printing, screen printing, stencil printing, slot die coating and a doctor blading technique. In a preferred embodiment, the conductive preparation is applied until the layer of the conductive preparation may have a thickness of 5 µm to 20 µm, preferably of 10 µm ± 2 µm. As used herein, the term "until" may include a single or a multiple application of the at least one material.

According to step d) of the present invention, the first side of the first substrate is laminated with the second side of the second substrate. In the embodiment in which the layer of a conductive preparation may, according to step c), be applied onto the third layer or the portion thereof, the first side of the first substrate can, preferably, be laminated onto the second side of the second substrate. Alternatively or in addition, in an embodiment in which the layer of the conductive preparation may, also according to step c), be applied onto the first side of the first substrate or the portion thereof, the second side of the second substrate can, preferably, be laminated onto the first side of the first substrate.

As generally used herein, the term "laminating" or any grammatical variation thereof relates to a process of joining at least two adjacent sides of at least two individual bodies in a planar fashion, whereby a permanently assembled object, which may also be denoted as a "sandwich", is generated as a result of this process. For this purpose, at least one of an adhesive, a pressure, or thermal energy may, especially, be applied to at least one adjacent side and/or to the preliminary assembled object. As used herein, the term "adjacent side" refers to a side of the individual body which is designated for being assembled to a further side of a further individual body. Further, the term "preliminary assembled object" relates to a spatial arrangement of the at least two individual bodies which enables an application of the at least one of the adhesive, the pressure, or the thermal energy in a manner that the permanently assembled object is obtained. As further used herein, the term "adhesive" refers to a material designated for, preferably permanently, assembling two adjacent sides of two individual bodies. For this purpose, a particular material, typically denoted as "adhesive" or "glue", may, specifically, be used for this purpose. However, according to the present invention, the conductive preparation, which is, according to step c), applied as a layer to the first side of the first substrate, the third layer, or a portion thereof, for forming an interjacent electrode is, in addition, already designated for assembling the respective surfaces in a permanent fashion and, thus, functions as an adhesive. As a result, the present invention offers the particular advantage that, in general, using a separate adhesive apart from using the conductive preparation is dispensable. However, a separate adhesive can, still, be used to assemble at least one portion of the respective surfaces which are designated for being maintained free from the conductive preparation.

In a particular embodiment, at least one further process may, in addition, be applied prior to performing the laminating process, especially, to improve at least one property of at least one adjacent side, wherein the at least one further process may, specifically, be selected from at least one of pre-processing or pre-coating; however, at last one further process may also be feasible. In a further particular embodiment, at least one further process may, in addition, be applied during or after performing the laminating process, especially, to improve the permanent assembly of the object, wherein the at least one further process may, specifically, be or comprise calendering; however, at last one further process may also be feasible. As generally used, the term "calendering" or any grammatical variation thereof relates to a process of finishing and/or smoothing the assembled object by applying a pressure using at least one hard pressure roller as denoted as "calender".

In a particular embodiment, the conductive preparation as provided during step c) may not be sticky at room temperature. As generally used, the term "room temperature" relates to a temperature of 20 °C to 25 °C. As further generally used, the term "sticky" relates to a property of material which support an adherence of the material on a substrate. In a preferred embodiment, in which the conductive preparation may be a solvent-free preparation, the solvent-free preparation can be heated to a temperature at which it may be transformed into a molten mass. Typically, this temperature may be 50 °C to 220 °C, preferably 60 °C to 120 °C, more preferred 80 °C to 100 °C, in particular, depending on the composition of the conductive preparation, in particular depending on the binder as comprised by the conductive preparation. The molten mass can be applied to the first side of the first substrate or to the third layer of the second substrate or a portion thereof, as desired. Thereinafter, the other of the first side of the first substrate or of the third layer of the second substrate may be laminated in accordance with step d) to the conductive preparation. Alternatively, the conductive preparation may comprise at least one solvent. Herein, the conductive preparation may, again, be applied to the first side of the first substrate or to the third layer or a portion thereof, as desired. Thereinafter, the other of the first side of the first substrate or of the third layer of the second substrate may be laminated in accordance with step d) to the conductive preparation, preferably, by using a temperature which may be sufficient to melt the conductive preparation at least partially.

In an alternative embodiment, the conductive preparation as provided during step c) may be sticky at room temperature. Herein, the conductive preparation may be applied to the first side of the first substrate or to the third layer or a portion thereof, as desired. Thereinafter, the other of the first side of the first substrate or of the third layer may be laminated in accordance with step d) to the conductive preparation, preferably, at room temperature.

As a result of the step d), the first side of the first substrate is permanently assembled with the second side of the second substrate by applying a lamination process, whereby, according to step e) of the present invention, the desired analyte sensor is, eventually, obtained. For this purpose, the binder as comprised by the layer of the conductive preparation as applied during step c) may, in particular, be functions as an adhesive in order to effect the joining as well as the permanently assembly of the first side of the first substrate with the second side of the second substrate. However, in contrast to known processes, a use of a particular adhesive may be dispensable.

In a particular embodiment, the permanently assembled object may comprise at least two individual analyte sensors. Herein, the individual analyte sensors can be separated from each other between the performance of steps d) and e) in a fashion that each of the individual analyte sensor is carried by a respective portion of the substrate. As generally used, the term "separating" or any grammatical variation thereof relates to a process of segmenting a joint object into at least two partitions. In particular, a separation process may be used for individualizing the analyte sensors from the joint permanently assembled object. For this purpose, at least one cutting process, especially selected from at least one of a dye cutting processes or a laser cutting process may be used. However, a different cutting process may be applicable.

Prior or, preferably, after step e), at least one further process may be applied to the analyte sensor, especially, to improve at least one property of the analyte sensor. Specifically, the finally assembled analyte sensor may be subject to a further treatment, especially selected from at least one of dip-coating, in particular for generating a membrane on a surface of an electrode designated as working electrode, or a chemical or electrochemical pre-treatment of one or more electrode in order to obtain a functional layer. However, a further process may also be feasible.

The analyte sensor as obtained during step e) comprises three individual electrodes which are arranged in a stack. In particular, an interlayer may be formed by the third layer and the layer of the conductive preparation. As a result, the interlayer may be an electrically conducting layer. Especially based on this property of the interlayer, the interlayer as generated by the method according to the present invention may, as particularly preferred, be or comprise a third electrode. As generally used, the term "interlayer" refers to a particular layer which is placed between two adjacent layers, preferably in an immediate fashion, especially without any further layer in between. As a result of laminating the first side of the first substrate with the second side of the second substrate according to step d), the analyte sensor as obtained in step e) has three individual electrodes which can be controlled and evaluated independently from each other.

In a preferred embodiment, the first electrode as attached to or comprised by the first layer may be or comprise a first working electrode, and the second electrode as attached to or comprised by the second layer may be or comprise a second working electrode, while the third electrode as formed or comprised by the interlayer may be counter electrode or, preferably, a combined counter/reference electrode. Herein, in a particular embodiment thereof, the first working electrode may be configured to monitor a concentration of a first analyte while the second working electrode may be configured to monitor a concentration of a second analyte being different from the first analyte, thereby providing "a two-in-one analytical sensor". By way of example, the first working electrode may be configured to monitor a concentration of glucose, and the second working electrode may be configured to monitor a concentration of ketones formed due to hyperglycemia, while the third electrode may, preferably, be a combined counter/reference electrode. In an alternative embodiment, the first electrode as attached to or comprised by the first layer may be or comprise a working electrode and the second electrode as attached to or comprised by the second layer may be or comprise a counter electrode, while the third electrode as formed or comprised by the interlayer may be or comprise a reference electrode. However, further kinds of embodiments with respect to the electrodes may also be feasible.

In a particularly preferred embodiment, each electrode may have a lateral extension of 1 mm to 25 mm, preferably of 2 mm to 20 mm, more preferred of 5 mm to 20 mm, and a thickness of 5 µm to 20 µm, preferably of 10 µm ± 2 µm. For the terms "lateral extension" and "thickness", reference may be made to the definitions given above. However, further embodiments of the electrodes may also be feasible.

In a further aspect of the present invention, an analyte sensor, in particular an analyte sensor obtainable by the method as described herein, is disclosed. Herein, the analyte sensor comprises:
- a first substrate having
   ∘ a first side, and
   ∘ a second side, wherein the second side has a first layer comprising a first conductive material;
- a second substrate having
   ∘ a first side, wherein the first side has a second layer comprising a second conductive material, and
   ∘ a second side, wherein second side has a third layer comprising a third conductive material;
- a layer of an conductive preparation placed onto at least one of the first side of the first substrate or the third layer or a portion thereof, wherein the conductive preparation comprises
   ∘ a plurality of conductive particles, and
   ∘ at least one polymeric binder;
      wherein the first side of the first substrate is laminated with the second side of the second substrate.

The analyte sensor as produced by the method as disclosed herein may, preferably, constitute a stack having a sensing end and a contacting end. At the sensing end of the stack, the laminate may comprise an interlayer formed by the third layer and the layer of the conductive preparation enclosed between the first substrate and the second substrate, wherein the interlayer may act as a combined counter/reference electrode, while the first layer and the second layer which are arranged at outer surfaces of the stack act as working electrodes. At the contacting end of the stack, all three layers can be contacted as electrodes at corresponding electrically conductive surfaces. As a result of the laminating of the first side of the first substrate with the second side of the second substrate, the analyte sensor has three individual electrodes which can be controlled and evaluated independently from each other.

For further details with regard to the analyte sensor, reference can be made to the description of the method for producing an analyte sensor above or below.

The method for producing an analyte sensor according to the present invention and the analyte sensor as obtained by applying this method exhibit particular advantages with respect to the prior art in that the analyte sensor as proposed herein. Preferably, the method for producing the analyte sensor can be performed in a cost-efficient fashion, thereby producing the analyte sensor in a reliable and fast manner. In particular, the method allows an individual tailoring of the analyte sensor according to the particular requirements of the desired operation. As a result, the analyte sensor as proposed herein, particularly, qualifies for a use in a continuous glucose monitoring system which demands a reliable and persistent operation over a long period of time. In contrast hereto, US 10,321,863 B2 and US 10,349,873 B2 disclose methods for producing an analyte sensor according to which the analyte sensor is generated in an elaborate, sophisticated and cost-intensive process using a layer-by layer approach.

As used herein, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. Herein, in most cases, when referring to the respective feature or element, the expressions "at least one" or "one or more" will not be repeated, non-withstanding the fact that the respective feature or element may be present once or more than once.

Further, as used herein, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

Summarizing, the following embodiments are potential embodiments of the present invention. However, other embodiments may also be feasible.
Embodiment 1. A method for producing an analyte sensor, the method comprising the steps of:
   a) providing a first substrate having
      ∘ a first side, and
      ∘ a second side, wherein the second side has a first layer comprising a first conductive material;
   b) providing a second substrate having
      ∘ a first side, wherein the first side has a second layer comprising a second conductive material, and
      ∘ a second side, wherein second side has a third layer comprising a third conductive material;
   c) applying a layer of an conductive preparation onto at least one of the first side of the first substrate or the third layer or a portion thereof, wherein the conductive preparation comprises
      ∘ a plurality of conductive particles, and
      ∘ at least one polymeric binder;
   d) laminating the first side of the first substrate with the second side of the second substrate; and
   e) obtaining the analyte sensor.
Embodiment 2. The method according to the preceding Embodiment, wherein the conductive particles comprise carbon, Ag, AgCl or Ag/AgCl.
Embodiment 3. The method according to any one of the preceding Embodiments, wherein the at least one polymeric binder is selected from at least one of a thermoplastic polyurethane (TPU), thermoplastic polyolefin (TPO), thermoplastic vulcanizates (TPV), thermoplastic copolyester (TPC), thermoplastic polyamides (TPA), a styrenic block copolymer (TPS), a copolymer of vinyl chloride (VC) and vinyl acetate (VAc) (Vinylester or vinylether), polyvinylether, polyvinylester, an acrylate, a methacrylate , an acrylic resin, a styrene acrylic resin, a vinyl acetal, a polyester, a polyether, acrylonitrile butadiene styrene (ABS), styrene-acrylonitrile resin (SAN), acrylonitrile styrene acrylate (ASA), styrene butadiene copolymer (SB), polystyrene (PS), polyethlene (PE), ethylene-vinyl acetate (EVA), polypropylene (PP), polybutylene (PB), polyisobutene (PIB), poylvinylchloride (PVC), polyvinyl alcohol (PVAL), or polylactic acid (PLA).
Embodiment 4. The method according to the preceding Embodiments, wherein the conductive preparation is not sticky at room temperature and wherein the conductive preparation is a solvent-free preparation, wherein the solvent-free preparation is heated until it is transformed into a molten mass, wherein the molten mass is applied to the first side of the first substrate or to the third layer or a portion thereof, wherein the other of the first side of the first substrate or of the third layer is laminated to the conductive preparation.
Embodiment 5. The method according to any one of the preceding Embodiments, wherein the conductive preparation is not sticky at room temperature and wherein the conductive preparation comprises at least one solvent, wherein the conductive preparation is applied to the first side of the first substrate or to the third layer or a portion thereof, wherein the other of the first side of the first substrate or of the third layer is laminated to the conductive preparation using a temperature which is sufficient to melt the conductive preparation.
Embodiment 6. The method according to any one of the preceding Embodiments, wherein the conductive preparation is sticky at room temperature, wherein the conductive preparation is applied to the first side of the first substrate or to the third layer or a portion thereof, wherein the other of the first side of the first substrate or of the third layer is laminated to the conductive preparation at room temperature.
Embodiment 7. The method according to any one of the preceding Embodiments, wherein the conductive preparation is applied until the layer of the conductive preparation has a thickness of 5 µm to 20 µm.
Embodiment 8. The method according to the preceding Embodiment, wherein the conductive preparation is applied until the layer of the conductive preparation has a thickness of 10 µm ± 2 µm.
Embodiment 9. The method according to any one of the preceding Embodiments, wherein the conductive preparation is applied only to a portion of at least one of the first side of the first substrate or the third layer, wherein an insulating layer, partially or completely, covers the remaining portion of the at least one of the first side of the first substrate or the third layer.
Embodiment 10. The method according to any one of the preceding Embodiments, wherein a first electrode is formed on the first layer.
Embodiment 11. The method according to any one of the preceding Embodiments, wherein the first layer is or comprises the first electrode.
Embodiment 12. The method according to any one of the two preceding Embodiments, wherein the first electrode is or comprises a first working electrode.
Embodiment 13. The method according to any one of the preceding Embodiments, wherein a second electrode is formed on the second layer.
Embodiment 14. The method according to any one of the preceding Embodiments, wherein the second layer is or comprises the second electrode.
Embodiment 15. The method according to any one of the two preceding Embodiments, wherein the second electrode is or comprises a second working electrode or a counter electrode.
Embodiment 16. The method according to any one of the preceding Embodiments, wherein an interlayer is formed by the third layer and the layer of the conductive preparation.
Embodiment 17. The method according to the preceding Embodiment, wherein the interlayer is or comprises a third electrode.
Embodiment 18. The method according to the preceding Embodiment, wherein the third electrode is or comprises a combined counter/reference electrode or a reference electrode.
Embodiment 19. The method according to any one of the nine preceding Embodiments, wherein the electrode has a lateral extension of 1 mm to 25 mm.
Embodiment 20. The method according to the preceding Embodiment, wherein the electrode has a lateral extension of 2 mm to 20 mm.
Embodiment 21. The method according to the preceding Embodiment, wherein the electrode has a lateral extension of 5 mm to 20 mm.
Embodiment 22. The method according to any one of the twelve preceding Embodiments, wherein the electrode has a thickness of 5 µm to 20 µm.
Embodiment 23. The method according to the preceding Embodiment, wherein the electrode has a thickness of 10 µm ± 2 µm.
Embodiment 24. The method according to any one of the fourteen preceding Embodiments, wherein the electrode comprises at least one conductive material.
Embodiment 25. The method according to the preceding Embodiment, wherein the at least one conductive material is selected from a noble metal or from an electrically conductive carbon material.
Embodiment 26. The method according to the preceding Embodiment, wherein the at least one conductive material is gold.
Embodiment 27. The method according to any one of the preceding Embodiments, wherein the substrate comprises at least one the electrically insulating material.
Embodiment 28. The method according to the preceding Embodiment, wherein the electrically insulating material is selected from polyethylene terephthalate (PET), polycarbonate (PC), polytetrafluoroethylene (PTFE), polyimide (PI), acrylonitrile butadiene styrene (ABS), styrene-acrylonitrile resin (SAN), acrylonitrile styrene acrylate (ASA), styrene butadiene copolymer (SB), polystyrene (PS), polyethlene (PE), ethylene-vinyl acetate (EVA), polypropylene (PP), polybutylene (PB), polyisobutene (PIB), poylvinylchloride (PVC), polyvinyl alcohol (PVAL), polylactic acid (PLA), polyamide (PA), polymethylmethacrylate (PMMA), polyoxymethylene (POM), polyphenylene sulfide (PPS), polysulfone (PSU), polyvinylidene fluoride (PVDF), fluorinated ethylene propylene (FEP), ethylene tetrafluoroethylene (ETFE), polyvinyl fluoride (PVF), polyethersilfone (PES), polyether ether ketone (PEEK,) perfluoroalkoxy alkane (PFA), or polyetherketoneetherketoneketone (PEKEKK).
Embodiment 29. The method according to any one of the preceding Embodiments, wherein the substrate has a lateral extension of 1 mm to 500 mm.
Embodiment 30. The method according to the preceding Embodiment, wherein the substrate has a lateral extension of 10 mm to 100 mm.
Embodiment 31. The method according to the preceding Embodiment, wherein the substrate has a lateral extension of 20 mm to 50 mm.
Embodiment 32. The method according to any one of the preceding Embodiments, wherein the substrate has a thickness of 10 µm to 100 µm.
Embodiment 33. The method according to the preceding Embodiment, wherein the substrate has a thickness of 20 µm to 80 µm.
Embodiment 34. The method according to the preceding Embodiment, wherein the substrate has a thickness of 50 µm to 80 µm.
Embodiment 35. The method according to any one of the preceding Embodiments, wherein a laminated substrate is obtained by step d).
Embodiment 36. The method according to the preceding Embodiment, wherein the laminated substrate is cut after step d) and prior to step e).
Embodiment 37. The method according to the preceding Embodiment, wherein the laminated substrate is cut by using at least one separation process.
Embodiment 38. The method according to the preceding Embodiment, wherein the at least one separation process is selected from at least one cutting process.
Embodiment 39. The method according to the preceding Embodiment, wherein the at least one cutting process is selected from at least one of a laser cutting process or a dye cutting process.
Embodiment 40. An analyte sensor, comprising
   - a first substrate having
      ∘ a first side, and
      ∘ a second side, wherein the second side has a first layer comprising a first conductive material;
   - a second substrate having
      ∘ a first side, wherein the first side has a second layer comprising a second conductive material, and
      ∘ a second side, wherein second side has a third layer comprising a third conductive material;
   - a layer of an conductive preparation placed onto at least one of the first side of the first substrate or the third layer or a portion thereof, wherein the conductive preparation comprises
      ∘ a plurality of conductive particles, and
      ∘ at least one polymeric binder;
         wherein the first side of the first substrate is laminated with the second side of the second substrate.
Embodiment 41. The analyte sensor according to the preceding Embodiment, wherein the analyte sensor is obtainable by the method according to any one of the preceding method Embodiments.
Embodiment 42. The analyte sensor according to any one of the preceding Embodiments referring to an analyte sensor, wherein the analyte sensor is a fully or partially implantable analyte sensor for continuously monitoring an analyte.
Embodiment 43. The analyte sensor according to any one of the preceding Embodiments referring to an analyte sensor, wherein the analyte sensor is an analyte sensor for continuously monitoring an analyte.
Embodiment 44. The analyte sensor according to any one of the preceding Embodiments referring to an analyte sensor, wherein the analyte sensor is an analyte sensor for a continuous measurement of the analyte in a subcutaneous tissue.
Embodiment 45. The analyte sensor according to any one of the preceding Embodiments referring to an analyte sensor, wherein the analyte sensor is an analyte sensor for a continuous measurement of the analyte in a body fluid.
Embodiment 46. The analyte sensor according to any one of the preceding Embodiments referring to an analyte sensor, wherein the analyte sensor is an analyte sensor for a continuous measurement of the analyte in an interstitial fluid.
Embodiment 47. The analyte sensor according to any one of the preceding Embodiments referring to an analyte sensor, wherein the analyte sensor is an analyte sensor for a continuous measurement of the analyte in blood.
Embodiment 48. The analyte sensor according to any one of the preceding Embodiments referring to an analyte sensor, wherein the analyte sensor is configured to convert the analyte into an electrically charged entity by using an enzyme.
Embodiment 49. The analyte sensor according to any one of the preceding Embodiments referring to an analyte sensor, wherein the analyte comprises glucose.
Embodiment 50. The analyte sensor according to the preceding Embodiment, wherein the analyte sensor is configured to convert glucose into an electrically charged entity by using an enzyme.
Embodiment 51. The analyte sensor according to the preceding Embodiment, wherein the enzyme is at least one of glucose oxidase or glucose dehydrogenase.

### Short description of the figures

Further details of the invention can be derived from the following disclosure of preferred embodiments. The features of the embodiments can be implemented in an isolated way or in any combination. The invention is not restricted to the embodiments. The embodiments are schematically depicted in the Figures. The Figures are not to scale. Identical reference numbers in the Figures refer to identical elements or functionally identical elements or elements corresponding to each other with regard to their functions. In the Figures:

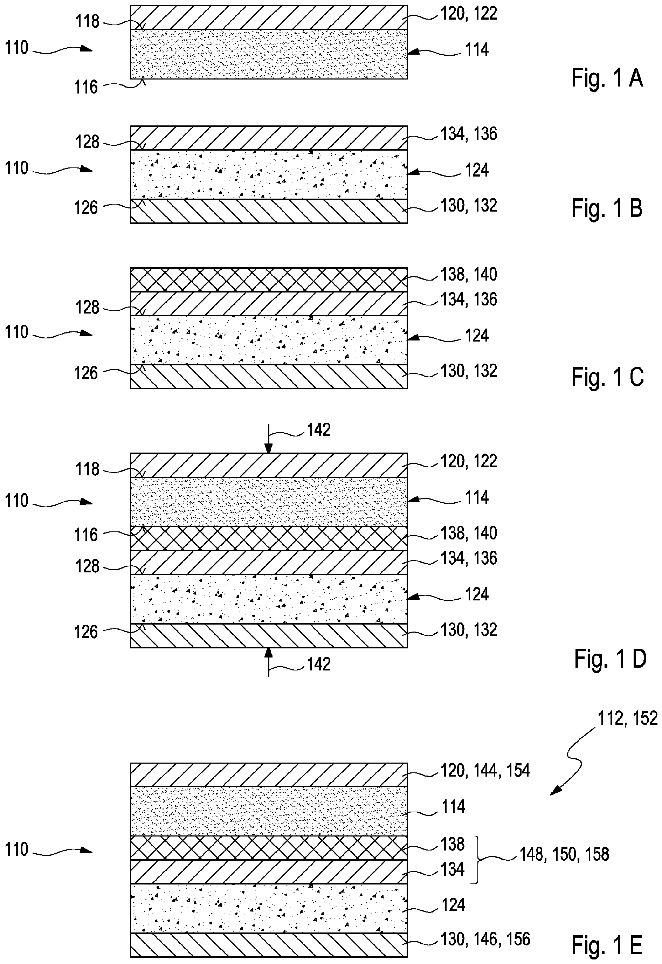

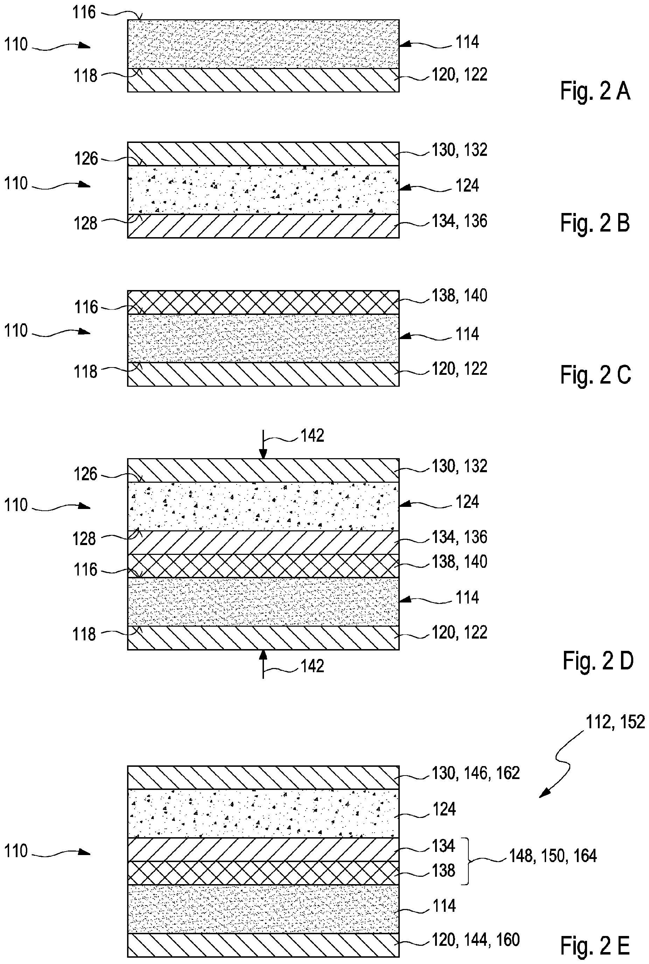

Figures 1 and 2 each schematically illustrates preferred embodiments of the method for producing an analyte sensor according to the present invention.

### Detailed description of the embodiments

Figures 1 and 2 each schematically illustrates a preferred embodiment of the method 110 for producing an analyte sensor 112, in particular a portion thereof, according to the present invention in cross-sectional views which are not to scale. The analyte sensor 112 may be a fully or partially implantable analyte sensor for continuously monitoring an analyte, in particular by performing a continuous measurement of the analyte in a subcutaneous tissue, preferably in a body fluid, especially in an interstitial fluid or in blood. For this purpose, the analyte sensor 112 may be configured to convert the analyte into an electrically charged entity by using an enzyme. Specifically, the analyte may comprises glucose, which may be converted into an electrically charged entity by using at least one of glucose oxidase (GOD) or glucose dehydrogenase (GHD) an the enzyme. As an alternative, the analyte sensor 112 may be configured to specifically detect two or more analytes in a concurrent or consecutive manner. Specifically, the analyte sensor 112 may be configured to detect glucose and, concurrently or consecutively, a further analyte, preferably selected from lactate or ketones, in a specific fashion. However, the analyte sensor 112 according to the present invention may also be applicable to other kinds of analytes as well as to other processes for monitoring an analyte. In the particular example of Figures 1 and 2, the implantable portion of the analyte sensor 112 as depicted there comprises an elongate analyte sensor; however, other forms of the analyte sensor 112 may also be feasible.

As illustrated in Figures 1a) and 2a), a first substrate 114 is provided in accordance with step a) of the method 110. Herein, the first substrate 114 has a first side 116 and a second side 118. Further, the second side 118 of the first substrate 114 has a first layer 120 which comprises a first conductive material 122, while the first side 116 of the first substrate 114 remains blank. Instead of having an uncoated insulating surface as depicted in Figures 1a) and 2a), the first side 116 of the first substrate 114 may, in an alternative embodiment not depicted here, comprise at least one electrically insulating layer. As further schematically shown in Figures 1a) and 2a), the first side 116 and the second side 118 of the first substrate 114 are arranged in an opposing fashion with respect to each other and the first substrate 114.

As illustrated in Figures 1b) and 2b), a second substrate 124 is provided in accordance with step b) of the method 110. Herein, the second substrate 124 has a first side 126 and a second side 128. Further, the first side 126 of the second substrate 124 has a second layer 130 which comprises a second conductive material 132 while the second side 128 of the second substrate 124 has a third layer 134 which comprises a third conductive material 136. As further schematically shown in Figures 1b) and 2b), the first side 126 and the second side 128 of the second substrate 124 are arranged in an opposing fashion with respect to each other and the second substrate 124.

In the particular examples of Figures 1 and 2, each of the first substrate 114 and the second substrate 124 is a planar substrate; however, each substrate 114, 124 may also have a different form. Further, each of the first substrate 114 and the second substrate 124 as illustrated there has an elongated shape, in particular a bar shape; however, other kinds of shapes may also be feasible. Preferably, each substrate 114, 124 may have a lateral extension of 1 mm to 500 mm, preferably of 10 mm to 100 mm, more preferred of 20 mm to 50 mm, and a thickness of 10 µm to 100 µm, preferably of 20 µm to 80 µm, more preferred of 50 µm to 80 µm; however, a different value for the lateral extension and/or the thickness may also be feasible.

In particular, each of the first substrate 114 and the second substrate 124 may be an electrically insulating substrate which may, preferably, comprise at least one electrically insulating material, especially to avoid unwanted currents between the second layer 120 and the third layer 134. Herein, the electrically insulating material may, preferably, be selected from polyethylene terephthalate (PET) or polycarbonate (PC); however, other kinds of electrically insulating materials, such as the insulating materials as indicated above, may also be feasible.

Further, each of the first conductive material 122 as comprised by the first layer 120, the second conductive material 132 as comprised by the second layer 130, and the third conductive material 136 as comprised by third layer 134 as shown in the particular examples of Figures 1 and 2 may, preferably, comprise an electrically conductive material. In particular, the conductive material may be selected from a noble metal, especially gold; or from an electrically conductive carbon material; however, further kinds of conductive materials may also be feasible. The layers 120, 130, 134 may, specifically, have an elongated shape, such as a strip shape or a bar shape; however, other kinds of shapes may also be feasible. In general, the layers 120, 132, 134 may, partially or completely, cover the respective sides 118, 126, 128 of the corresponding substrate 114, 124. In a preferred embodiment, in which the layers 120, 132, 134 may only partially cover a portion of the respective sides 118, 126, 128 of the corresponding substrate 114, 124, an insulating layer (not depicted here) may, partially or completely, cover the remaining portion of the corresponding substrate 114, 124. In particular, the layers 120, 132, 134 may, preferably, be produced, prior to steps a) and b), using an additive process by applying, especially by depositing, the conductive material to the respective sides 118, 126, 128 of the corresponding substrate 114, 124; however, a further process of producing the layers 120, 132, 134 may be feasible. In particular, the additive process may be selected from at least one process as indicated above, wherein sputtering may particularly be preferred.

As illustrated in Figure 1c), a layer 138 of a conductive preparation 140 is, according to step c), applied onto the third layer 134 or a portion thereof. In an alternative embodiment as illustrated in Figure 2c), the layer 138 of the conductive preparation 140 is applied onto the first side 116 of the first substrate 114 or a portion thereof. It is emphasized here that selecting either the procedure as shown in Figure 1c) or the procedure as shown in Figure 2c) may not result in a difference in the analyte sensor as obtained by the method 110.

The conductive preparation 140 comprises at least one first component and at least one second component, wherein the at least one first component is or comprises a plurality of conductive particles, wherein the at least one second component is or comprises at least one polymeric binder. However, further types of components may also be conceivable, in particular at least one solvent. In a particularly preferred embodiment, the conductive particles comprise carbon, Ag, AgCl or Ag/AgCl. In a further preferred embodiment, the at least one polymeric binder may be selected from at least one of a thermoplastic polyurethane (TPU) or an acrylate, wherein the thermoplastic polyurethane (TPU) may particularly be preferred. For further kinds of suitable polymeric binders, reference can be made to the description above. The layer 138 of the conductive preparation 140 may be applied by any suitable additive process which may be selected from at least one process as indicated above, wherein a deposition process may particularly be preferred. In a particularly preferred embodiment, the conductive preparation 140 may applied onto the third layer 134 or a portion thereof, or on the first side 116 of the first substrate 114 or a portion thereof, respectively, until the layer 138 of the conductive preparation 140 may have a thickness of 5 µm to 20 µm, preferably of 10 µm ± 2 µm.

As illustrated in Figures 1d) and 2d), the first side 116 of the first substrate 114 is, according to step d), laminated with the second side 128 of the second substrate 124. In the embodiment as shown in Figure 1d) in which the layer 138 of a conductive preparation 140 is applied onto the third layer 134 or the portion thereof, the first side 116 of the first substrate 114 is laminated onto the second side 128 of the second substrate 124. In the alternative embodiment as illustrated in Figure 2d), in which the layer 138 of the conductive preparation 140 is applied onto the first side 116b of the first substrate 114 or the portion thereof, the second side 128 of the second substrate 114 is laminated onto the first side 116 of the first substrate 114 in a laminating process indicated by arrows carrying reference sign 142. Independently of the embodiment as selected during step d), the process of joining the adjacent sides 116, 128 of the two individual substrates 114, 124 results in a permanently assembled object, also denoted as a "sandwich", which constitutes the desired analytical sensor 144 as illustrated in Figures 1e) and 2e).

In a particular embodiment, at least one further process may, in addition, be applied prior to the laminating process 142, especially, to improve at least one property of at least one of the sides 116, 128 as affected by the laminating process 142. Herein, the at least one further process may, specifically, be selected from at least one of pre-processing or pre-coating; however, at last one further process may also be feasible. In a further particular embodiment, at least one further process may, in addition, be applied during or after the laminating process 142, especially, to improve the permanent assembly of the analyte sensor 112. Herein, the at least one further process may, specifically, be or comprise finishing and/or smoothing the analyte sensor 112 by applying a calendering process; however, at last one further process may also be feasible.

In a particular embodiment, TPU which is not sticky at room temperature can be used as the polymeric binder in the conductive preparation 140. In a first embodiment, solvent-free TPU and a solvent-free TPU-based Ag/AgCl paste, which are solid at room temperature, are heated up to a temperature of 80 °C to 100 °C to reach a liquid state. Both molten masses are transferred to one of the substrates 114, 124, especially, by using a slit die. While the coating is still hot and sticky, the other one of the substrates 114, 124 is applied to the conductive preparation 140, whereby the laminated analyte sensor 112 is obtained.

Optionally, calendering can be used. In an alternative embodiment, both materials the TPU and the TPU-based Ag/AgCl paste are solutions. Both solutions are transferred to one of the substrates 114 124, especially, by using a slit die. Both solutions are dried in order to solidify, whereby the solvent is removed, at least to a large extent. In order to generate the laminated analyte sensor 112, hot laminating can be applied using a temperature which is sufficient to melt both solutions. In a still further embodiment, the conductive preparation may comprise acrylate-based Ag/AgCl, which is sticky also at room temperature, such that cold laminating can be used. However, further embodiments may also be feasible.

As schematically illustrated in Figures 1e) and 2e), the analyte sensor 112 as obtained during step e) comprises:
- a first electrode 144 which corresponds here to the first layer 120;
- a second electrode 146 which corresponds here to the first layer 130; and
- a third electrode 148 which corresponds here to an interlayer 150 which is formed by the third layer 134 and the layer 138 of the conductive preparation 140 in a fashion that the interlayer 150 is electrically conductive,

Herein, the electrodes 144, 146, 148 are arranged in a stack 152 as schematically depicted in Figures 1e) and 2e). In a further embodiment (not depicted here), the first electrode 144 may, alternatively or in addition, be formed as an additional conductive layer which may be attached to the first layer 120 and/or the second electrode 146 may, alternatively or in addition, be formed as a further additional conductive layer which may be attached to the second layer 130. Herein, each electrode 144, 146, 148 may have a lateral extension of 1 mm to 25 mm, preferably of 2 mm to 20 mm, more preferred of 5 mm to 20 mm, and a thickness of 5 µm to 20 µm, preferably of 10 µm ± 2 µm. However, further embodiments of the electrodes may also be feasible.

In a preferred embodiment as schematically shown in Figure 1e), the first electrode 144 corresponding to the first layer 120 may be or comprise a first working electrode 154 and the second electrode 146 corresponding to the second layer 130 may be or comprise a second working electrode 156 while the third electrode 148 as provided by the interlayer 150 may, preferably, be or comprise a combined counter/reference electrode 158.

In an alternative embodiment as schematically shown in Figure 2e), the first electrode 144 corresponding to the first layer 120 may be or comprise the working electrode 160 and the second electrode 146 corresponding to the second layer 130 may be or comprise a counter electrode 162, while the third electrode 148 as provided by the interlayer 150 may be a reference electrode 164. However, further kinds of embodiments with respect to the electrodes 144, 146, 148 may also be feasible, in particular a further embodiment in which the electrodes 144, 146, 148 as used in the stack 152 of Figure 2e) may comprise the same assignment as the corresponding electrodes 144, 146, 148 as used in the stack 152 of Figure 1e), or vice-versa.

### List of reference numbers

- 110: method for producing an analyte sensor
- 112: analyte sensor
- 114: first substrate
- 116: first side
- 118: second side
- 120: first layer
- 122: first conductive material
- 124: second substrate
- 126: first side
- 128: second side
- 130: second layer
- 132: second conductive material
- 134: third layer
- 136: third conductive material
- 138: layer
- 140: conductive preparation
- 142: laminating process
- 144: first electrode
- 146: second electrode
- 148: third electrode
- 150: interlayer
- 152: stack
- 154: first working electrode
- 156: second working electrode
- 158: counter/reference electrode
- 160: working electrode
- 162: counter electrode
- 164: reference electrode

## Claims

1. A method (110) for producing an analyte sensor (112), the method (110) comprising the steps of:
a) providing a first substrate (114) having
∘ a first side (116), and
∘ a second side (118), wherein the second side (118) has a first layer (120) comprising a first conductive material (122);
b) providing a second substrate (124) having
∘ a first side (126), wherein the first side (126) has a second layer (130) comprising a second conductive material (132), and
∘ a second side (128), wherein second side (128) has a third layer (134) comprising a third conductive material (136);
c) applying a layer (138) of a conductive preparation (140) onto at least one of the first side (116) of the first substrate (114) or the third layer (134) or a portion thereof, wherein the conductive preparation (140) comprises
∘ a plurality of conductive particles, and
∘ at least one polymeric binder;
d) laminating the first side (116) of the first substrate (114) with the second side (128) of the second substrate (124); and
e) obtaining the analyte sensor (112).

2. The method (110) according to claim 1, wherein the conductive particles comprise carbon, Ag, AgCl or Ag/AgCl.

3. The method (110) according to any one of claims 1 or 2, wherein the at least one polymeric binder is selected from at least one of a thermoplastic polyurethane and an acrylate.

4. The method (110) according to any one of the claims 1 to 3, wherein the conductive preparation (140) is applied until the layer (138) of the conductive preparation (140) has a thickness of 5 µm to 20 µm.

5. The method (110) according to any one of claims 1 to 4, wherein a first electrode (144) is formed on the first layer (120), or wherein the first layer (120) is or comprises the first electrode (144).

6. The method (110) according to claim 5, wherein the first electrode (144) is or comprises a first working electrode (154, 160).

7. The method (110) according to any one of claims 1 to 6, wherein a second electrode (146) is formed on the second layer (130), or wherein the second layer (130) is or comprises the second electrode (146).

8. The method (110) according to claim 7, wherein the second electrode (146) is or comprises a second working electrode (156) or a counter electrode (162).

9. The method (110) according to any one of claims 1 to 8, wherein an interlayer (150) is formed by the third layer (134) and the layer (138) of the conductive preparation (140).

10. The method (110) according to claim 9, wherein the interlayer (150) is or comprises a third electrode (148).

11. The method (110) according to claim 10, wherein the third electrode (148) is or comprises a combined counter/reference electrode (158) or a reference electrode (164).

12. The method (110) according to any one of claims 1 to 11, wherein a laminated substrate is obtained by step d), wherein the laminated substrate is cut after step d) and prior to step e).

13. The method (110) according to claim 12, wherein the laminated substrate is cut by using at least one separation process, wherein the at least one separation process is selected from at least one of a laser cutting process and a dye cutting process.

14. An analyte sensor (112) obtainable by a method (110) according to any one of claims 1 to 13.

15. A use of an analyte sensor (112) according to claim 14 in a continuous glucose monitoring system.
